# EUROPÄISCHE PATENTANMELDUNG

(11) **EP 3 462 184 A1**
(43) Veröffentlichungstag der Anmeldung: **03.04.2019**
(21) Anmeldenummer: 17193781.6
(22) Anmeldetag: 28.09.2017
(51) Int. Cl.: G01R 15/18, G01R 15/20, A61F 9/06, B23K 9/095, B23K 9/10, B23K 9/32

(54) **ERFASSUNGSVORRICHTUNG FÜR EINE AKTIVE BLENDSCHUTZVORRICHTUNG**

(71) Anmelder: optrel Holding AG, 9050 Appenzeil (CH)
(72) Erfinder: SCHREIBER, Olaf, 9470 Buchs/SG (CH); HOFER KRANER, Ramon, 9100 Herisau (CH); PITTORINO, Tindaro, 9470 Buchs/SG (CH)
(74) Vertreter: Daub, Thomas

(57) **Zusammenfassung**

Die Erfindung geht aus von einer Erfassungsvorrichtung für eine aktive Blendschutzvorrichtung (12), mit einer Erfassungseinheit (14), welche zu einer direkten oder indirekten Erfassung zumindest eines Schweißparameters (A) eines Schweißgeräts (16) vorgesehen ist, und mit zumindest einer Kommunikationseinheit (18), welche zu einer Übertragung des zumindest einen Schweißparameters (A) an die aktive Blendschutzvorrichtung (12) vorgesehen ist.

Es wird vorgeschlagen, dass die Erfassungseinheit (14) zumindest einen Stromsensor (20) aufweist, welcher zu einer Erfassung des zumindest einen Schweißparameters (A) des Schweißgeräts (16) dazu vorgesehen ist, variabel an ein Stromkabel (22) des Schweißgeräts (16) befestigt zu werden.

## Beschreibung

### Stand der Technik

Die Erfindung betrifft eine Erfassungsvorrichtung für eine aktive Blendschutzvorrichtung.

Es ist bereits eine Erfassungsvorrichtung für eine aktive Blendschutzvorrichtung, mit einer Erfassungseinheit, welche zu einer direkten oder indirekten Erfassung zumindest eines Schweißparameters eines Schweißgeräts vorgesehen ist, und mit zumindest einer Kommunikationseinheit, welche zu einer Übertragung des zumindest einen Schweißparameters an die aktive Blendschutzvorrichtung vorgesehen ist, vorgeschlagen worden.

Die Aufgabe der Erfindung besteht insbesondere darin, eine gattungsgemäße Vorrichtung mit verbesserten Eigenschaften hinsichtlich einer Reaktionszeit und Zuverlässigkeit bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Erfassungsvorrichtung für eine aktive Blendschutzvorrichtung, mit einer Erfassungseinheit, welche zu einer direkten oder indirekten Erfassung zumindest eines Schweißparameters eines Schweißgeräts vorgesehen ist, und mit zumindest einer Kommunikationseinheit, welche zu einer Übertragung des zumindest einen Schweißparameters an die aktive Blendschutzvorrichtung vorgesehen ist.

Es wird vorgeschlagen, dass die Erfassungseinheit zumindest einen Stromsensor aufweist, welcher zu einer Erfassung des zumindest einen Schweißparameters des Schweißgeräts dazu vorgesehen ist, variabel an einem Stromkabel des Schweißgeräts befestigt zu werden. Unter einer "aktiven Blendschutzvorrichtung" soll in diesem Zusammenhang insbesondere eine Blendschutzvorrichtung mit einem aktiven optischen Blendschutzfilter verstanden werden. Dabei soll unter einer "Blendschutzvorrichtung" in diesem Zusammenhang insbesondere eine Vorrichtung verstanden werden, welche zu einem Schutz eines Benutzers vor zu großer Helligkeit und/oder vor Funken vorgesehen ist. Vorzugsweise soll darunter insbesondere eine Vorrichtung verstanden werden, welche zu einem Schutz von Augen und/oder einem Gesichtsbereich eines Benutzers während eines Schweiß- und/oder Schleifprozesses dient. Bevorzugt soll darunter insbesondere eine Blendschutzvorrichtung verstanden werden, welche insbesondere zu einem Schutz der Augen eines Benutzers zumindest während eines Schweißprozesses dient. Es sind verschiedene, einem Fachmann als sinnvoll erscheinende Ausgestaltungen einer Blendschutzvorrichtung denkbar, wie beispielsweise als Schweißhelm, -schirm, -maske und/oder -schild. Ferner soll dabei unter einem "aktiven optischen Blendschutzfilter" insbesondere ein optischer Filter verstanden werden, welcher insbesondere ein Schutzglas und/oder ein Kunststoffschutzglas bildet. Vorzugsweise soll darunter insbesondere ein optischer Filter verstanden werden, dessen Lichtdurchlässigkeit einstellbar ausgeführt ist. Bevorzugt soll darunter insbesondere ein optischer Schweißschutzfilter mit einer automatischen Verdunkelung verstanden werden. Besonders bevorzugt weist der Blendschutzfilter zumindest eine in der Transmission schaltbare Flüssigkristallebene auf. Es sind verschiedene, einem Fachmann als sinnvoll erscheinende Ausgestaltungen des optischen Blendschutzfilters denkbar, insbesondere soll darunter jedoch ein ADF, auch "automatic darkening filter" oder "automatischer Schweißerschutzfilter" genannt, verstanden werden. Des Weiteren soll unter einer "Erfassungseinheit" in diesem Zusammenhang insbesondere eine Einheit verstanden werden, welche zu einer direkten oder indirekten Erfassung zumindest eines Schweißparameters eines Schweißgeräts vorgesehen ist. Die Erfassungseinheit kann dabei insbesondere sowohl zu einer direkten Erfassung des Schweißparameters vorgesehen sein, als auch dazu vorgesehen sein, durch Erfassung alternativer Kenngrößen auf den zumindest einen Parameter zu schließen. Vorzugsweise umfasst die Erfassungseinheit zumindest einen Sensor. Dabei soll unter einem "Sensor" in diesem Zusammenhang insbesondere ein Element verstanden werden, das dazu vorgesehen ist, zumindest eine Kenngröße und/oder eine physikalische Eigenschaft aufzunehmen, wobei die Aufnahme aktiv, wie insbesondere durch Erzeugen und Aussenden eines elektrischen Messsignals, und/oder passiv, wie insbesondere durch eine Erfassung von Eigenschaftsänderungen eines Sensorbauteils, stattfinden kann. Es sind verschiedene, dem Fachmann als sinnvoll erscheinende Sensoren denkbar. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Unter einer "Kommunikationseinheit" soll in diesem Zusammenhang insbesondere eine Einheit verstanden werden, welche zu einer Bereitstellung einer, insbesondere kabellosen, Kommunikation mit der aktiven Blendschutzvorrichtung vorgesehen ist. Vorzugsweise weist die Kommunikationseinheit zu einer Kommunikation mit der aktiven Blendschutzvorrichtung zumindest eine Schnittstelle auf. Vorzugsweise soll unter einer Kommunikationseinheit insbesondere eine Einheit verstanden werden, welche zu einem Austausch von Daten vorgesehen ist. Insbesondere weist die Kommunikationseinheit zumindest einen Informationseingang und zumindest einen Informationsausgang auf. Vorzugsweise weist die Kommunikationseinheit zumindest zwei Informationseingänge und zumindest zwei Informationsausgänge auf, wobei jeweils zumindest ein Informationseingang und zumindest ein Informationsausgang zu einer Verbindung mit einem physischen System, insbesondere dem externen Schweißgerät, vorgesehen sind. Besonders bevorzugt soll darunter eine Schnittstelle zwischen zumindest zwei physischen Systemen, wie insbesondere zwischen der aktiven Blendschutzvorrichtung und der Erfassungsvorrichtung, verstanden werden. Es sind verschiedene, dem Fachmann als sinnvoll erscheinende Kommunikationseinheiten denkbar, insbesondere soll darunter jedoch eine drahtlose Schnittstelle, wie beispielsweise Bluetooth, WLAN, Zigbee, NFC, RFID, GSM, LTE oder UMTS, und/oder eine drahtgebundene Schnittstelle, wie beispielsweise ein USB-Anschluss, eine Canbus-Schnittstelle, eine RS485 Schnittstelle, eine Ethernet-Schnittstelle, eine optische Schnittstelle, eine KNX-Schnittstelle und/oder eine Powerline-Schnittstelle, verstanden werden.

Ferner soll in diesem Zusammenhang unter einem "Stromsensor" insbesondere ein Sensor verstanden werden, welcher dazu vorgesehen ist, direkt, wie insbesondere mittels elektrischer Kontakte, oder indirekt, wie beispielsweise durch eine Kabelisolierung hindurch, wie insbesondere mittels einer Spule und/oder einem Hall-Sensor, auf eine Stromstärke und/oder eine Stromänderung zu schließen. Es sind verschiedene, einem Fachmann als sinnvoll erscheinende Stromsensoren denkbar. Darunter, dass der "Stromsensor dazu vorgesehen ist, variabel an ein Stromkabel des Schweißgeräts befestigt zu werden", soll in diesem Zusammenhang insbesondere verstanden werden, dass der Stromsensor dazu vorgesehen ist, unabhängig von einem Schweißgerät an ein Stromkabel eines Schweißgeräts befestigt zu werden. Vorzugsweise kann der Stromsensor an einer beliebigen Position an dem Stromkabel des Schweißgeräts befestigt werden. Bevorzugt soll darunter insbesondere verstanden werden, dass der Stromsensor dazu vorgesehen ist, optional und unabhängig von dem Schweißgerät an das Stromkabel des Schweißgeräts befestigt zu werden. Vorzugsweise soll darunter insbesondere verstanden werden, dass der Stromsensor kein Teil des Schweigeräts ist. Vorzugsweise kann der Stromsensor beispielsweise zwischen einem Stecker des Schweißgeräts und einer Steckdose angeordnet werden. Bei einer entsprechenden Ausgestaltung wäre insbesondere denkbar, dass die Erfassungsvorrichtung bei einer fehlenden oder fehlerhaften Kommunikation mit der aktiven Blendschutzvorrichtung eine Energiezufuhr des Schweißgeräts unterbricht. Hierdurch kann insbesondere sinnvoll ein Bediener geschützt werden.

Durch die erfindungsgemäß Ausgestaltung der Erfassungsvorrichtung kann insbesondere eine schnelle und zuverlässige Erfassung des zumindest einen Schweißparameters des Schweißgeräts erreicht werden. Vorzugsweise kann dadurch insbesondere zu einer Frühabdunkelung des aktiven Blendschutzfilters zuverlässig und insbesondere unabhängig von dem Schweißgerät ein Strom des Schweißgeräts erfasst werden. Insbesondere kann der Strom des Schweißgeräts, welcher zu einer Erzeugung eines Schweisslichtbogens benötigt wird, meist schon früher detektiert werden, als ein Schweisslichtbogen optisch erfasst werden kann, da der Schweisslichtbogen bei den meisten Verfahren eine gewisse Zeit benötigt, um sich aufzubauen. Erfolgt also in dieser Zeit eine Triggerung des aktiven Blendschutzfilters, kann bereits während eines Aufbaus des Schweisslichtbogens genau auf die zur optischen Detektion benötigte Helligkeit des Schweisslichtbogens abgedunkelt werden. Hierdurch kann insbesondere, vorzugsweise gegenüber einer optischen Detektion, eine vorteilhaft schnelle Abdunkelung der aktiven Blendschutzvorrichtung erreicht werden. Die Erfassung eines entstehenden Schweisslichtbogens erfolgt insbesondere durch die Detektion einer starken Stromänderung.

Ferner wird vorgeschlagen, dass der Stromsensor zu einer Erfassung des zumindest einen Schweißparameters des Schweißgeräts dazu vorgesehen ist, elektrisch kontaktlos, variabel an das Stromkabel des Schweißgeräts befestigt zu werden. Vorzugsweise ist der Stromsensor zu einer Erfassung des zumindest einen Schweißparameters des Schweißgeräts dazu vorgesehen, elektrisch kontaktlos, das Stromkabel des Schweißgeräts umgreifend, an das Stromkabel befestigt zu werden. Unter "elektrisch kontaktlos" soll in diesem Zusammenhang insbesondere verstanden werden, dass der Stromsensor einen stromführenden Teil des Stromkabels nicht berührt. Vorzugsweise weist der Stromsensor keinen elektrischen Kontakt zu einer Kontaktierung eines stromführenden Teils des Stromkabels auf. Dadurch kann insbesondere eine vorteilhaft variable Anordnung des Stromsensors an dem Stromkabel erreicht werden. Es kann insbesondere erreicht werden, dass der Stromsensor beliebig und komfortabel positioniert werden kann.

Des Weiteren wird vorgeschlagen, dass der zumindest eine Stromsensor der Erfassungseinheit zumindest ein erstes Sensorelement aufweist, welches zu einer Erfassung einer Stromänderung in dem Stromkabel des Schweißgeräts vorgesehen ist. Vorzugsweise erfasst das erste Sensorelement eine reine Stromänderung in dem Stromkabel. Dadurch kann insbesondere vorteilhaft zuverlässig eine Aktivierung des Schweißgeräts erfasst werden. Es kann insbesondere vorteilhaft schnell und zuverlässig eine Aktivierung des Schweißgeräts erfasst werden.

Zudem wird vorgeschlagen, dass das zumindest eine erste Sensorelement von einer Rogowskispule gebildet ist. Unter einer "Rogowskispule" soll insbesondere eine toroidförmige Luftspule verstanden werden. Vorzugsweise soll darunter insbesondere eine Spule frei von einem ferromagnetischen Kern verstanden werden. Mittels der Rogowskispule sind extrem kurze Detektionsvorgänge möglich, wie insbesondere im Bereich von 1 µs. Die Spule erkennt insbesondere nur Stromänderungen und schlägt somit beim Einschalten des Stroms aus. Bei konstantem Strom gibt die Spule keinen Wert aus. Hierdurch kann eine vorteilhaft schnelle Detektion einer Stromänderung erreicht werden. Es kann insbesondere unabhängig von einer Größenordnung der Stromänderung zuverlässig die Stromänderung detektiert werden.

Es wird ferner vorgeschlagen, dass der zumindest eine Stromsensor der Erfassungseinheit zumindest ein zweites Sensorelement aufweist, welches zu einer Erfassung eines absoluten Stromwerts in dem Stromkabel des Schweißgeräts vorgesehen ist. Vorzugsweise ist das zweite Sensorelement zu einer Erfassung eines absoluten Stromwerts und damit insbesondere auch zu einer Erfassung anhaltender Stromwerte in dem Stromkabel des Schweißgeräts vorgesehen. Dadurch kann insbesondere vorteilhaft zuverlässig festgestellt werden, ob ein Schweißprozess aktiv ist. Vorzugsweise kann dadurch insbesondere ein Sensorelement bereitgestellt werden, mittels welchem ein Schweißende erfasst werden kann und mittels welchem festgelegt werden kann, ab welchem Stromgrenzwert der Blendenschutz wieder geöffnet werden soll.

Es wird weiter vorgeschlagen, dass das zumindest eine zweite Sensorelement von einem Hallsensor gebildet ist. Vorzugsweise dient der Hallsensor dazu, einen anhaltenden absoluten Stromwert zu erkennen, um abhängig davon einen Shutter der aktiven Blendschutzvorrichtung abgedunkelt zu halten. Ein Hallsensor alleine wäre jedoch insbesondere zu langsam, um den Initialstrompuls zu erkennen. Zu einer Erkennung des Initialstrompulses wird daher insbesondere die Rogowskispule genutzt. Dadurch kann insbesondere eine vorteilhaft zuverlässige Erfassung eines absoluten Stromwerts, insbesonder elektrisch kontaktlos, erreicht werden. Der Hallsensor wird insbesondere zu einer Sicherstellung einer zuverlässigen Detektion eines anhaltenden Gleichstroms verwendet.

Ferner wird vorgeschlagen, dass die zumindest eine Kommunikationseinheit zu einer kabellosen Übertragung des zumindest einen Schweißparameters an die aktive Blendschutzvorrichtung vorgesehen ist. Vorzugsweise soll eine Übertragung dabei in weniger als 20 µs erfolgen. Hierdurch kann insbesondere auf ein Verbindungskabel zwischen der aktiven Blendschutzvorrichtung und dem Schweißgerät bzw. der Erfassungsvorrichtung verzichtet werden. Es kann insbesondere eine vorteilhaft komfortable Nutzung gewährleistet werden.

Des Weiteren wird vorgeschlagen, dass die zumindest eine Kommunikationseinheit zu einer schnellen Datenübertragung zumindest einen Differentialverstärker, zumindest einen aktiven Gleichrichter und/oder zumindest einen Komparator aufweist. Durch die Kopplung von verschiedenen Verarbeitungsstufen wird insbesondere ein eindeutiges Signal erzeugt. Dies gibt entweder ein "Schweißen aktiv" oder "inaktiv" auf einem Signalausgang, insbesondere einem TTL-Signalausgang, aus. Das Signal wird vorzugsweise anschließend mittels schneller Datenübertragung kabellos an den aktiven Blendschutzfilter gesendet. Die Signalaufbereitung wird insbesondere durch einen Differentialverstärker, einen aktiven Gleichrichter und abschliessend durch einen Komparator sichergestellt. Unter einem "Differentialverstärker" soll in diesem Zusammenhang insbesondere ein elektronisches Bauteil verstanden werden, welches zu einer Verstärkung zumindest eines Eingangssignals vorgesehen ist. Vorzugsweise soll darunter insbesondere ein Verstärker mit zumindest zwei Eingängen verstanden werden. Der aktive Gleichrichter dient insbesondere der Gleichrichtung eines verstärkten Ausgangssignals zumindest eines Sensorelements des Stromsensors, insbesondere der Rogowskispule, welches bezogen zu einer Referenzspannung in Abhängigkeit von der Stromrichtung positiv oder negativ ausfallen kann. Durch die aktive Gleichrichtung kann insbesondere erreicht werden, dass der Schweißstrom richtungsunabhängig erfasst werden kann. Der Komfort wird erhöht, da der Sensor keine Vorzugsrichtung bei der Montage um das Schweisskabel aufweisen muss. Die gesamte analoge Signalaufbereitung, insbesondere in Verbindung mit der Rogowskispule, kann eine sehr schnelle und zuverlässige Erfassung des Schweißbeginns sicherstellen.

Es wird ferner vorgeschlagen, dass die Erfassungsvorrichtung zumindest ein zumindest teilweise mehrteiliges Gehäuse aufweist, welches dazu vorgesehen ist, insbesondere werkzeuglos um das Stromkabel des Schweißgeräts herum montiert zu werden. Vorzugsweise besteht das Gehäuse aus zwei Hälften oder auch einem Verbund und kann um das Kabel herummontiert werden. Bevorzugt bildet das Gehäuse eine Art Schelle, welche werkzeuglos entfernt und montiert werden kann. Es wäre auch denkbar, eine Schleife oder dergleichen um das Kabel zu binden. Es muss insbesondere sichergestellt sein, dass der Stromkreis des ersten Sensorelements geschlossen werden kann. Dadurch kann insbesondere eine einfache und schnelle Montage der Erfassungsvorrichtung erreicht werden. Hierdurch kann insbesondere eine komfortable Bedienung der Erfassungsvorrichtung ermöglicht werden.

Es wird weiter vorgeschlagen, dass die Erfassungsvorrichtung zumindest eine mehrteilige Platine aufweist, die zumindest zwei, zumindest teilweise getrennte Platinenelemente aufweist, welche dazu vorgesehen sind, um das Stromkabel des Schweißgeräts herum montiert zu werden, wobei das zumindest eine erste Sensorelement zumindest teilweise auf das erste Platinenelement und zumindest teilweise auf das zweite Platinenelement aufgebracht ist. Vorzugsweise ist die Platine als geteilte Platine ausgebildet, welche mit SMD bestückbaren Induktivitäten, insbesondere einzelnen Luftspulen der Rogowskispule, bestückt ist. Dadurch kann insbesondere eine einfache und schnelle Montage der Erfassungsvorrichtung erreicht werden. Ferner kann eine vorteilhafte Funktionalität des Stromsensors erreicht werden.

Ferner wird ein System mit einer aktiven Blendschutzvorrichtung und mit der Erfassungsvorrichtung vorgeschlagen.

Des Weiteren wird ein Verfahren zu einem Betrieb der Erfassungsvorrichtung vorgeschlagen.

Die erfindungsgemäße Erfassungsvorrichtung, das System sowie das Verfahren sollen hierbei nicht auf die oben beschriebene Anwendung und Ausführungsform beschränkt sein. Insbesondere können die erfindungsgemäße Erfassungsvorrichtung, das System sowie das Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein System mit einer aktiven Blendschutzvorrichtung und mit einer erfindungsgemäßen Erfassungsvorrichtung und ein Schweißgerät in einer schematischen Darstellung,
- Fig. 2: die erfindungsgemäße Erfassungsvorrichtung mit einer Erfassungseinheit und mit einer Kommunikationseinheit in einer schematischen Vorderansicht,
- Fig. 3: die erfindungsgemäße Erfassungsvorrichtung mit der Erfassungseinheit und mit der Kommunikationseinheit in einer schematischen Rückansicht,
- Fig. 4: die erfindungsgemäße Erfassungsvorrichtung während einer Montage an einem Stromkabel des Schweißgeräts in einer stark schematischen Darstellung,
- Fig. 5: die erfindungsgemäße Erfassungsvorrichtung nach einer Montage an dem Stromkabel des Schweißgeräts in einer stark schematischen Darstellung,
- Fig. 6: ein erstes Sensorelement eines Stromsensors der Erfassungseinheit der erfindungsgemäßen Erfassungsvorrichtung in einer schematischen Darstellung,
- Fig. 7: ein schematisches Diagramm einer Reaktionszeit der erfindungsgemäßen Erfassungsvorrichtung gegenüber einer herkömmlichen optischen Erfassung und
- Fig. 8: ein schematisches Ablaufdiagramm eines Verfahrens zu einem Betrieb der erfindungsgemäßen Erfassungsvorrichtung.

### Beschreibung des Ausführungsbeispiels

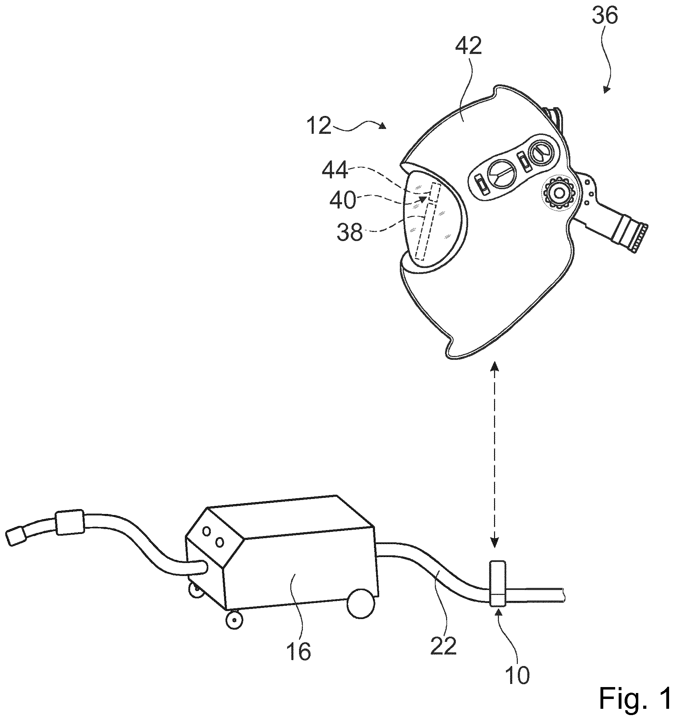

Figur 1 zeigt eine aktiven Blendschutzvorrichtung 12, eine Erfassungsvorrichtung 10 und ein Schweißgerät 16. Das Schweißgerät 16 ist von einem elektrisch betriebenen Schweißgerät gebildet. Das Schweißgerät 16 ist von einem Lichtbogenschweißgerät gebildet. Grundsätzlich wäre jedoch auch eine andere, einem Fachmann als sinnvoll erscheinende Ausbildung des Schweißgeräts 16 denkbar.

Die Blendschutzvorrichtung 12 und die Erfassungsvorrichtung 10 bilden ein System 36 aus. Die Blendschutzvorrichtung 12 ist dazu vorgesehen, während eines Betriebs von einem Bediener auf dem Kopf getragen zu werden. Die Blendschutzvorrichtung 12 ist von einem Schweißhelm gebildet. Grundsätzlich wäre jedoch auch eine andere, einem Fachmann als sinnvoll erscheinende Ausbildung der Blendschutzvorrichtung 12 denkbar. Die Erfassungsvorrichtung 10 ist von der Blendschutzvorrichtung 12 getrennt ausgebildet.

Die Blendschutzvorrichtung 12 weist einen optischen Blendschutzfilter 38 auf. Der Blendschutzfilter 38 ist dazu vorgesehen, abhängig von einem Arbeitszustand und einer davon abhängigen Ansteuerung eine Durchlässigkeit zu verändern. Der optische Blendschutzfilter 38 ist von einem elektrooptischen Filter gebildet. Der optische Blendschutzfilter 38 ist von einem automatic darkening filter, kurz ADF, gebildet. Der optische Blendschutzfilter 38 besteht aus mehreren Schichten. Der optische Blendschutzfilter 38 ist als ein Mehrschichtverbund ausgebildet. Die Flüssigkristallschicht des Blendschutzfilters 38 wird während eines Betriebs von der Recheneinheit 40 abgedunkelt, wenn ein Schweißverfahren erfasst wird. Der Blendschutzfilter 38 weist eine rechteckige Grundform auf.

Ferner weist die Blendschutzvorrichtung 12 eine Schildeinheit 42 auf. Der Blendschutzfilter 38 ist fest in der Schildeinheit 42 aufgenommen. Der Blendschutzfilter 38 ist positionsfest in der Schildeinheit 42 aufgenommen. Die Blendschutzvorrichtung 12 weist ferner eine Vorsatzscheibe auf. Die Vorsatzscheibe ist mit der Schildeinheit 42 über nicht weiter sichtbare Rastelemente verbunden. Die Vorsatzscheibe ist transparent ausgebildet. Die Vorsatzscheibe ist zu einem Schutz des Blendschutzfilters 38 vorgesehen. Die Vorsatzscheibe verdeckt den Blendschutzfilter 38 von außen.

Des Weiteren weist die Blendschutzvorrichtung 12 eine Kommunikationseinheit 44 auf. Die Kommunikationseinheit 44 ist zu einer Kommunikation mit der von der Blendschutzvorrichtung 12 getrennten Erfassungsvorrichtung 10 vorgesehen. Die Kommunikationseinheit 44 ist in die Recheneinheit 40 integriert. Die Kommunikationseinheit 44 bildet ein Teil der Recheneinheit 40. Grundsätzlich wäre jedoch auch eine separate Ausbildung der Kommunikationseinheit 44 denkbar. Über die Kommunikationseinheit 44 kann die Blendschutzvorrichtung 12 Signale zu einer Abdunkelung des Blendschutzfilters 38 empfangen. Über die Kommunikationseinheit 44 kann die Blendschutzvorrichtung 12 mittels der Erfassungsvorrichtung 10 erfasste Schweißparameter A des Schweißgeräts 16 empfangen. Die Schweißparameter A des Schweißgeräts 16 werden von der Erfassungsvorrichtung 10 an die Blendschutzvorrichtung 12 übertragen. Die Recheneinheit 40 der Blendschutzvorrichtung 12 ist zu einer Auswertung der Schweißparameter A des Schweißgeräts 16 vorgesehen. Ferner ist die Recheneinheit 40 dazu vorgesehen, abhängig von den Schweißparametern A des Schweißgeräts 16 den Blendschutzfilter 38 abzudunkeln. Hierbei wäre es insbesondere denkbar, dass die Recheneinheit 40 den Blendschutzfilter 38 bei einem Ausbleiben eines Signals von der Erfassungsvorrichtung 10 den Blendschutzfilter 38 automatisch abdunkelt. Hierdurch könnte bei einem Ausbleiben eines Signals von der Erfassungsvorrichtung 10, beispielsweise aufgrund einer zu großen Entfernung zwischen der Blendschutzvorrichtung 12 und der Erfassungsvorrichtung 10, eine Schädigung des Bedieners ausgeschlossen werden. Zudem oder alternativ wäre jedoch auch denkbar, dass die Recheneinheit 40 einen zusätzlichen Sensor zu einer Erfassung eines Lichtbogens des Schweißgeräts 16 aufweist, mittels welchem der Blendschutzfilter 38 ebenfalls abgedunkelt werden kann.

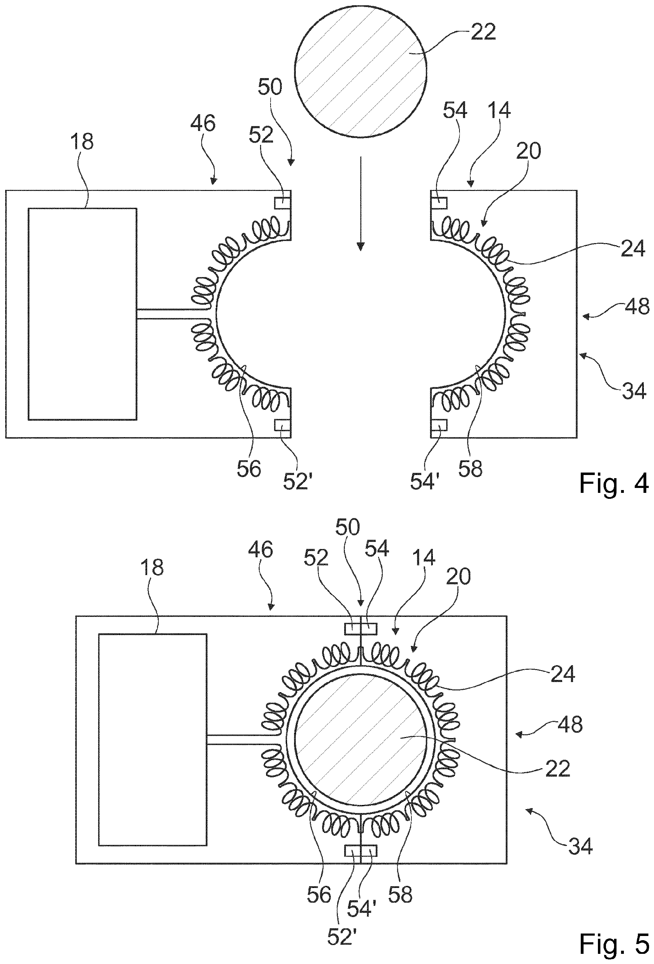

Die Erfassungsvorrichtung 10 ist für die aktive Blendschutzvorrichtung 12 vorgesehen. Die Erfassungsvorrichtung 10 bildet ein Zusatzbauteil für die Blendschutzvorrichtung 12. Die Erfassungsvorrichtung 10 und die Blendschutzvorrichtung 12 sind zu einer gemeinsamen Benutzung vorgesehen. Die Erfassungsvorrichtung 10 weist eine Erfassungseinheit 14 auf. Die Erfassungseinheit 14 ist zu einer direkten oder indirekten Erfassung zumindest eines Schweißparameters A des Schweißgeräts 16 vorgesehen. Die Erfassungseinheit 14 ist zu einer indirekten Erfassung eines Stroms des Schweißgeräts 16 vorgesehen. Die Erfassungseinheit 14 ist zu einer Erfassung eines zu dem Schweißgerät 16 fließenden Stroms vorgesehen. Die Erfassungseinheit 14 weist einen Stromsensor 20 auf. Der Stromsensor 20 ist zu einer Erfassung des Schweißparameters A des Schweißgeräts 16 vorgesehen. Der Stromsensor 20 ist zu einer Erfassung eines von einem Strom gebildeten Schweißparameters A des Schweißgeräts 16 vorgesehen. Der Stromsensor 20 ist zu einer Erfassung des Schweißparameters A des Schweißgeräts 16 dazu vorgesehen, variabel an ein Stromkabel 22 des Schweißgeräts 16 befestigt zu werden. Das Stromkabel 22 des Schweißgeräts 16 ist von einem Brenner- und/oder Massekabel gebildet. Der Stromsensor 20 ist zu einer Erfassung des zumindest einen Schweißparameters A des Schweißgeräts 16 dazu vorgesehen, elektrisch kontaktlos, variabel an das Stromkabel 22 des Schweißgeräts 16 befestigt zu werden. Der Stromsensor 20 ist in einem Betrieb variabel an ein Stromkabel 22 des Schweißgeräts 16 befestigt. Die gesamte Erfassungsvorrichtung 10 ist in einem Betrieb variabel an ein Stromkabel 22 des Schweißgeräts 16 befestigt. Die Erfassungsvorrichtung 10 umgreift dazu das Stromkabel 22 des Schweißgeräts 16. Die Erfassungsvorrichtung 10 wird dazu um das Stromkabel 22 des Schweißgeräts 16 herum befestigt. Die Erfassungsvorrichtung 10 bildet eine Schelle aus. Die Erfassungsvorrichtung 10 kann an einer beliebigen Position an dem Stromkabel 22 des Schweißgeräts 16 sowie an einem beliebigen Schweißgerät 16 befestigt werden. Die Erfassungsvorrichtung 10 ist unabhängig von einem Schweißgerät 16 funktionsfähig und kann insbesondere an Schweißgeräte 16 verschiedener Hersteller befestigt werden (Figuren 1, 5).

Die Erfassungsvorrichtung 10 weist ein mehrteiliges Gehäuse 34 auf. Die Erfassungsvorrichtung 10 weist ein zweiteiliges Gehäuse 34 auf. Das Gehäuse 34 nimmt die Erfassungseinheit 14 der Erfassungsvorrichtung 10 auf. Das Gehäuse 34 ist dazu vorgesehen, werkzeuglos um das Stromkabel 22 des Schweißgeräts 16 herum montiert zu werden. Das Gehäuse 34 weist eine erste Gehäusehälfte 46 und eine zweite Gehäusehälfte 48 auf. Die Gehäusehälften 46, 48 weisen jeweils auf einander zugewandten Seiten eine Aussparung 56, 58 für das Stromkabel 22 auf. Die Aussparungen 56, 58 der Gehäusehälften 46, 48 sind jeweils halbkreisförmig ausgebildet. Die Aussparungen 56, 58 bilden gemeinsam eine kreisförmige Aussparung. Die Gehäusehälften 46, 48 sind über eine Verbindungseinheit 50 miteinander verbindbar. Die Verbindungseinheit 50 umfasst zwei erste Verbindungselemente 52, 52', welche fest mit der ersten Gehäusehälfte 46 verbunden sind, und zwei zweite Verbindungselemente 54, 54', welche fest mit der zweiten Gehäusehälfte 48 verbunden sind. Die ersten und zweiten Verbindungselemente 52, 52', 54, 54' werden zu einer Verbindung der Gehäusehälften 46, 48 miteinander verschraubt. Grundsätzlich wäre jedoch auch eine andere, einem Fachmann als sinnvoll erscheinende Verbindungstechnik denkbar, wie beispielsweise magnetisch. Zu einer Montage können die Gehäusehälften 46, 48 der Erfassungsvorrichtung 10 daher getrennt und um das Stromkabel 22 herum wieder geschlossen werden (Figuren 4, 5).

Ferner weist die Erfassungsvorrichtung 10 eine mehrteilige Platine 35 auf. Die Platine 35 ist zweiteilig ausgebildet. Die Platine 35 weist zwei getrennte Platinenelemente 37, 37' auf. Die Platinenelemente 37, 37' sind korrespondierend zu den Gehäusehälften 46, 48 ausgebildet. Das erste Platinenelement 37 ist in der ersten Gehäusehälfte 46 angeordnet. Das zweite Platinenelement 37' ist in der zweiten Gehäusehälfte 48 angeordnet. Die Platinenelemente 37, 37' sind dazu vorgesehen, um das Stromkabel 22 des Schweißgeräts 16 herum montiert zu werden. Die Platinenelemente 37, 37' weisen dazu zu den Aussparungen 56, 58 der Gehäusehälften 46, 48 korrespondierende Aussparungen auf.

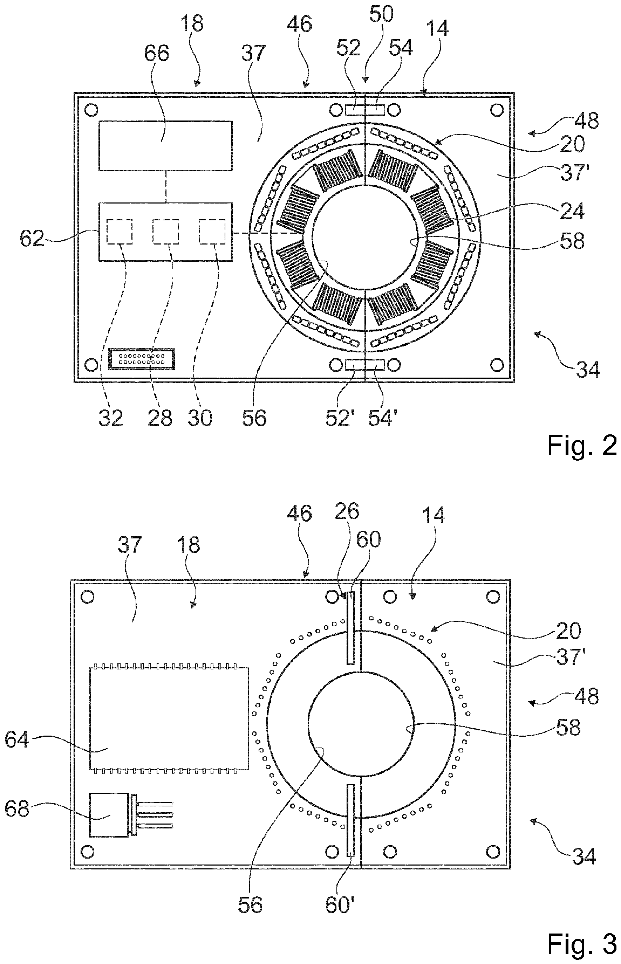

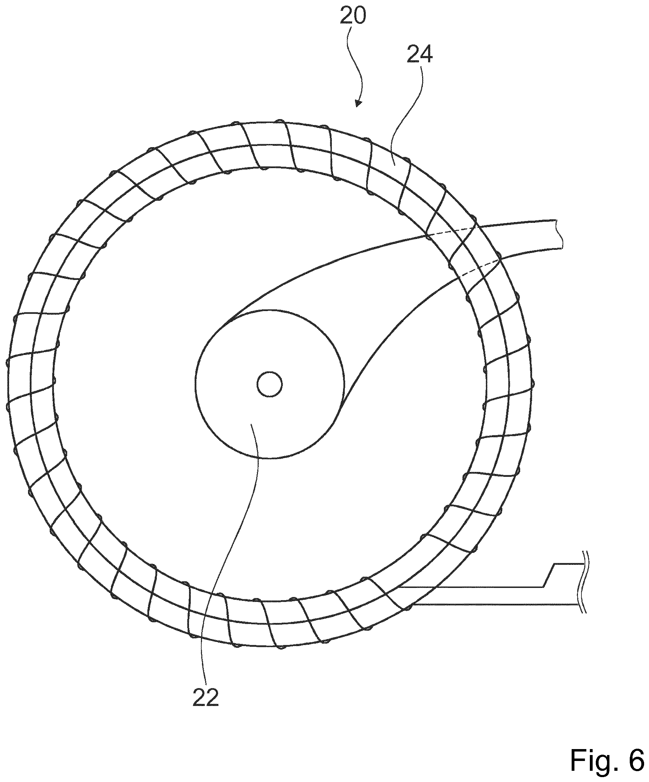

Ferner weist der Stromsensor 20 der Erfassungseinheit 14 ein erstes Sensorelement 24 auf. Das erste Sensorelement 24 ist zu einer Erfassung einer Stromänderung in dem Stromkabel 22 des Schweißgeräts 16 vorgesehen. Das erste Sensorelement 24 umgreift in einem Betrieb das Stromkabel 22 des Schweißgeräts 16. Das erste Sensorelement 24 des Stromsensor 20 ist um die Aussparungen 56, 58 der Gehäusehälften 46, 48 angeordnet. Das erste Sensorelement 24 ist zweiteilig ausgebildet. Eine Hälfte des ersten Sensorelements 24 ist in der ersten Gehäusehälfte 46 angeordnet und eine zweite Hälfte des ersten Sensorelements 24 ist in der zweiten Gehäusehälfte 48 angeordnet. Die Hälften des ersten Sensorelements 24 sind in einem verbundenen Zustand der Gehäusehälften 46, 48 ebenfalls verbunden. Die Verbindung der beiden Hälften erfolgt über eine robuste Kontaktierung, wie beispielsweise mittels einer Steckverbindung, einer Magnetverbindung und/oder einer losen Berührung. Die Hälften des ersten Sensorelements 24 sind in einem verbundenen Zustand der Gehäusehälften 46, 48 elektrisch verbunden. Das erste Sensorelement 24 ist von einer Rogowskispule gebildet. Das erste Sensorelement 24 ist von einer Rogowskispule gebildet, welche sich in einem Betrieb ringförmig um das Stromkabel 22 erstreckt. Das erste Sensorelement 24 ist von einer Rogowskispule gebildet, welche sich ringförmig um die Aussparungen 56, 58 erstreckt. Vorzugsweise ist die Rogowskispule aus mehreren Teilspulen zusammengesetzt. Das erste Sensorelement 24 ist teilweise auf das erste Platinenelement 37 und teilweise auf das zweite Platinenelement 37' aufgebracht. Das erste Sensorelement 24 besteht aus mehreren Luftspulen, mit welchen die Platinenelemente 37, 37' bestückt sind. Das erste Sensorelement 24 besteht aus mehreren SMD bestückbaren Luftspulen (Figuren 2, 4, 6).

Des Weiteren weist der Stromsensor 20 der Erfassungseinheit 14 ein zweites Sensorelement 26 auf. Das zweite Sensorelement 26 ist zu einer Erfassung eines absoluten Stromwerts in dem Stromkabel 22 des Schweißgeräts 16 vorgesehen. Mit dem zweiten Sensorelement 26 ist daher insbesondere auch ein anhaltender Stromwert messbar. Das zweite Sensorelement 26 weist zwei Messelemente 60, 60' auf, welche auf gegenüberliegenden Seiten der Aussparungen 56, 58 der Gehäusehälften 46, 48 angeordnet sind, Die Messelemente 60, 60' sind in einem Betrieb auf gegenüberliegenden Seiten des Stromkabels 22 angeordnet. Die Messelemente 60, 60' des zweiten Sensorelements 26 sind in der ersten Gehäusehälfte 46 des Gehäuses 34 angeordnet. Grundsätzlich wäre jedoch auch eine andere, einem Fachmann als sinnvoll erscheinende Anordnung des zweiten Sensorelements 26 denkbar. Das zweite Sensorelement 26 ist von einem Hallsensor gebildet. Die Messelemente 60, 60' des zweiten Sensorelements 26 sind jeweils von Hallsensorboards gebildet. Grundsätzlich wäre jedoch auch eine andere, einem Fachmann als sinnvoll erscheinende Ausbildung des zweiten Sensorelements 26 denkbar (Figur 3).

Die Erfassungsvorrichtung 10 weist ferner eine Kommunikationseinheit 18 auf. Die Kommunikationseinheit 18 ist zu einer Übertragung des Schweißparameters A an die aktive Blendschutzvorrichtung 12 vorgesehen. Die Kommunikationseinheit 18 der Erfassungsvorrichtung 10 ist zu einer Kommunikation mit der Kommunikationseinheit 44 der Blendschutzvorrichtung 12 vorgesehen. Die Kommunikationseinheit 18 der Erfassungsvorrichtung 10 ist zu einer Übertragung des Schweißparameters A an die Kommunikationseinheit 44 der Blendschutzvorrichtung 12 vorgesehen. Die Kommunikationseinheit 18 ist zu einer kabellosen Übertragung des Schweißparameters A an die aktive Blendschutzvorrichtung 12 vorgesehen. Die Kommunikationseinheit 18 weist zu einer schnellen Datenübertragung einen Differentialverstärker 28, einen aktiven Gleichrichter 30 und/oder einen Komparator 32 auf. Die Kommunikationseinheit 18 weist zu einer schnellen Datenübertragung einen Differentialverstärker 28, einen aktiven Gleichrichter 30 und einen Komparator 32 auf. Die Kommunikationseinheit 18 weist eine Signalverarbeitungseinheit 62 auf. Die Signalverarbeitungseinheit 62 ist von einer analogen Signalverarbeitung gebildet. Die Signalverarbeitungseinheit 62 ist zu einer Verbreitung der Daten der Erfassungseinheit 14 vorgesehen. Die Signalverarbeitungseinheit 62 ist zu einer Verbreitung der Daten der Erfassungseinheit 14 vor einem Versenden vorgesehen. Die Signalverarbeitungseinheit 62 ist direkt mit der Erfassungseinheit 14 gekoppelt. Die Signalverarbeitungseinheit 62 umfasst den Differentialverstärker 28, den aktiven Gleichrichter 30 und den Komparator 32. Ferner weist die Kommunikationseinheit 18 eine Sendeeinheit 64 auf. Die Sendeeinheit 64 ist zu einer Datenübertragung der Daten der Signalverarbeitungseinheit 62 vorgesehen. Die Sendeeinheit 64 ist zu einer direkten Übertragung der Daten an die Kommunikationseinheit 44 der Blendschutzvorrichtung 12 vorgesehen. Die Sendeeinheit 64 ist von einem Funkmodul gebildet. Die Sendeeinheit 64 ist von einem RF-Modul gebildet. Die Sendeeinheit 64 ist von einem System-on-a-Chip-RF-Modul, kurz RF-SoC-Modul, gebildet. Des Weiteren weist die Kommunikationseinheit 18 ein Netzteil 66 auf. Das Netzteil 66 dient zu einer Energieversorgung. Das Netzteil 66 ist über einen Batterieanschluss 68 mit einem nicht weiter sichtbaren Energiespeicher verbunden (Figuren 2, 3).

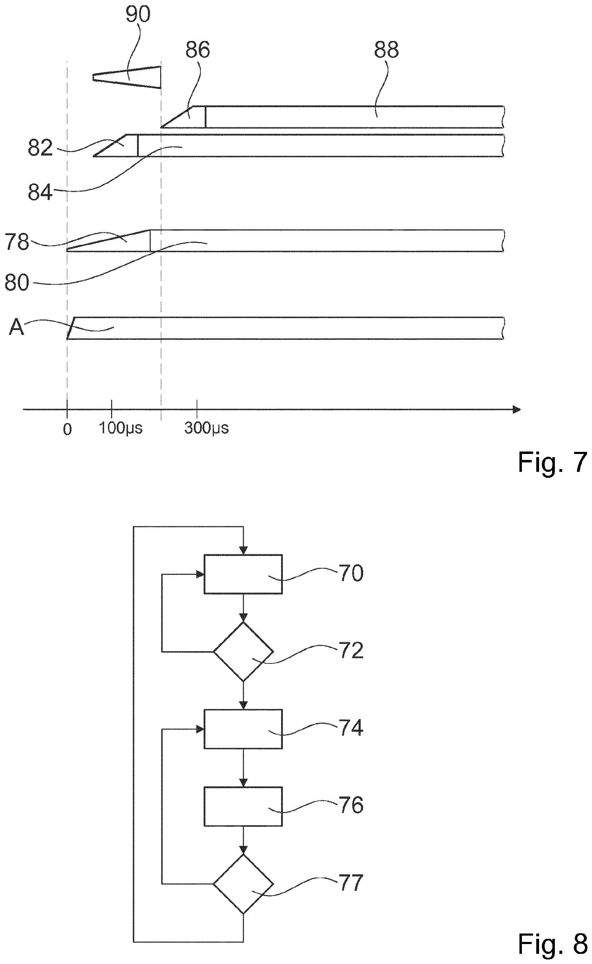

Figur 8 zeigt ein schematisches Ablaufdiagramm eines Verfahrens zu einem Betrieb der erfindungsgemäßen Erfassungsvorrichtung 10. Bei dem Verfahren wird in einem ersten Verfahrensschritt 70 mittels der Erfassungseinheit 14 eine in dem Stromkabel 22 des Schweißgeräts 16 vorliegende Stromänderung gemessen. In dem ersten Verfahrensschritt 70 wird mit dem ersten Sensorelement 24 einschließlich der Signalverarbeitungseinheit 62 ständig eine Stromänderung erfasst. Der erste Verfahrensschritt 70 bildet einen Ruhezustand aus. In dem ersten Verfahrensschritt 70 wird über die Kommunikationseinheit 18 kontinuierlich ein Statussignal "schweißen inaktiv" an die Blendschutzvorrichtung 12 übertragen. Durch die ständige Übertragung eines Status kann eine Verbindung zwischen der Erfassungsvorrichtung 10 und der Blendschutzvorrichtung 12 überwacht werden. Anschließend wird in einer ersten Verzweigung 72 überwacht, ob eine Stromänderung in dem Stromkabel 22 einen festgelegten Grenzwert überschreitet. Bleibt eine Änderung des Stroms unterhalb des Grenzwerts, wird der erste Verfahrensschritt 70 wiederholt. Übersteigt die erfasste Stromänderung den Grenzwert, wird dies in einem zweiten Verfahrensschritt 74 sofort an die Kommunikationseinheit 18 übertragen, welche sofort ein Statussignal "schweißen aktiv" an die Blendschutzvorrichtung 12 überträgt. Eine Übertragung des neuen Statussignals erfolgt dabei in weniger als 50 µs. Anschließend wird die Blendschutzvorrichtung 12 in einem dritten Verfahrensschritt 76 sofort abgedunkelt. Darauffolgend wird in einer zweiten Verzweigung 77 der absolute Stromwert in dem Stromkabel 22 erfasst. Die Erfassung des absoluten Stromwerts erfolgt mittels des zweiten Sensorelements 26. Wenn der absolute Stromwert einen Grenzwert überschreitet, wird der zweite Verfahrensschritt 74 wiederholt und es wird kontinuierlich das Statussignal "schweißen aktiv" an die Blendschutzvorrichtung 12 gesendet. Die Blendschutzvorrichtung 12 bleibt daher abgedunkelt. Wird der Grenzwert nicht überschritten, wird der erste Verfahrensschritt 70 wiederholt. Die Erfassungseinheit geht daher wieder in den Ruhezustand und sendet das Statussignal "schweißen inaktiv". Die Blendenschutzvorrichtung 12 wird wieder geöffnet. Nach der Übertragung öffnet die Blendenschutzvorrichtung 12 daher wieder. Das Öffnen der Blendschutzvorrichtung 12 ist dabei insbesondere nicht so zeitkritisch und kann nach einigen Millisekunden erfolgen. Damit ist das ungünstigere Zeitverhalten des zweiten Sensorelements 26 für die Erfassung des Schweissendes ausreichend. Da die Funkübertragung immer kontinuierlich erfolgt, ist neben der zeitnahen Übertragung eines neuen Zustands auch eine Überwachung der Funkverbindung möglich.

Figur 7 zeigt ein schematisches Diagramm der Reaktionszeit der Erfassungsvorrichtung 10 im Vergleich zu einem herkömmlichen Detektionsverfahren, bei welchem der Lichtbogen des Schweißgeräts 16 optisch erfasst wird. In dem Diagramm ist der von dem Strom des Schweißgeräts 16 gebildete Schweißparameter A als Balken dargestellt. Ferner ist in dem Diagramm als weiterer Balken 78 ein Aufbau des Lichtbogens sowie als ein daran anschließender Balken 80 der Lichtbogen selbst aufgetragen. Des Weiteren ist als weiterer Balken 82 ein Schließvorgang des Blendschutzfilters 38 sowie daran anschließend als weiterer Balken 84 ein Schließzustand des Blendschutzfilters 38 bei der Erfassungsvorrichtung 10 aufgetragen. Zudem ist als weiterer Balken 86 ein Schließvorgang des Blendschutzfilters 38 sowie daran anschließend als weiterer Balken 88 ein Schließzustand des Blendschutzfilters 38 bei einem herkömmlichen Detektionsverfahren aufgetragen. Da der Strom meist schon früher detektierbar ist und da der Schweisslichtbogen bei den meisten Verfahren eine gewisse Zeit benötigt um sich aufzubauen, kann eine Triggerung des Schweissschutzshutters bereits während des Bogenaufbaus erfolgen, sodass eine beschleunigte Abdunklung erreicht werden kann. Ein Balken 90 stellt die verbesserte Schließgeschwindigkeit beispielhaft dar.

## Patentansprüche

1. Erfassungsvorrichtung für eine aktive Blendschutzvorrichtung (12), mit einer Erfassungseinheit (14), welche zu einer direkten oder indirekten Erfassung zumindest eines Schweißparameters (A) eines Schweißgeräts (16) vorgesehen ist, und mit zumindest einer Kommunikationseinheit (18), welche zu einer Übertragung des zumindest einen Schweißparameters (A) an die aktive Blendschutzvorrichtung (12) vorgesehen ist,
**dadurch gekennzeichnet, dass**
die Erfassungseinheit (14) zumindest einen Stromsensor (20) aufweist, welcher zu einer Erfassung des zumindest einen Schweißparameters (A) des Schweißgeräts (16) dazu vorgesehen ist, variabel an ein Stromkabel (22) des Schweißgeräts (16) befestigt zu werden.

2. Erfassungsvorrichtung nach Anspruch 1,
**dadurch gekennzeichnet, dass**
der Stromsensor (20) zu einer Erfassung des zumindest einen Schweißparameters (A) des Schweißgeräts (16) dazu vorgesehen ist, elektrisch kontaktlos, variabel an das Stromkabel (22) des Schweißgeräts (16) befestigt zu werden.

3. Erfassungsvorrichtung nach Anspruch 1 oder 2,
**dadurch gekennzeichnet, dass**
der zumindest eine Stromsensor (20) der Erfassungseinheit (14) zumindest ein erstes Sensorelement (24) aufweist, welches zu einer Erfassung einer Stromänderung in dem Stromkabel (22) des Schweißgeräts (16) vorgesehen ist.

4. Erfassungsvorrichtung nach Anspruch 3,
**dadurch gekennzeichnet, dass**
das zumindest eine erste Sensorelement (24) von einer Rogowskispule gebildet ist.

5. Erfassungsvorrichtung nach einem der vorhergehenden Ansprüche,
**dadurch gekennzeichnet, dass**
der zumindest eine Stromsensor (20) der Erfassungseinheit (14) zumindest ein zweites Sensorelement (26) aufweist, welches zu einer Erfassung eines absoluten Stromwerts in dem Stromkabel (22) des Schweißgeräts (16) vorgesehen ist.

6. Erfassungsvorrichtung nach Anspruch 5,
**dadurch gekennzeichnet, dass**
das zumindest eine zweite Sensorelement (26) von einem Hallsensor gebildet ist.

7. Erfassungsvorrichtung nach einem der vorhergehenden Ansprüche,
**dadurch gekennzeichnet, dass**
die zumindest eine Kommunikationseinheit (18) zu einer kabellosen Übertragung des zumindest einen Schweißparameters (A) an die aktive Blendschutzvorrichtung (12) vorgesehen ist.

8. Erfassungsvorrichtung nach einem der vorhergehenden Ansprüche,
**dadurch gekennzeichnet, dass**
die zumindest eine Kommunikationseinheit (18) zu einer schnellen Datenübertragung zumindest einen Differentialverstärker (28), zumindest einen aktiven Gleichrichter (30) und/oder zumindest einen Komparator (32) aufweist.

9. Erfassungsvorrichtung nach einem der vorhergehenden Ansprüche,
**gekennzeichnet durch**
zumindest ein zumindest teilweise mehrteiliges Gehäuse (34), welches dazu vorgesehen ist, insbesondere werkzeuglos, um das Stromkabel (22) des Schweißgeräts (16) herum montiert zu werden.

10. Erfassungsvorrichtung zumindest nach Anspruch 4,
**gekennzeichnet durch**
zumindest eine mehrteilige Platine (35), die zumindest zwei, zumindest teilweise getrennte Platinenelemente (37, 37') aufweist, welche dazu vorgesehen sind um das Stromkabel (22) des Schweißgeräts (16) herum montiert zu werden, wobei das zumindest eine erste Sensorelement (24) zumindest teilweise auf das erste Platinenelement (37) und zumindest teilweise auf das zweite Platinenelement (37') aufgebracht ist.

11. System mit einer aktiven Blendschutzvorrichtung (12) und mit einer Erfassungsvorrichtung (10) nach einem der vorhergehenden Ansprüche.

12. Verfahren zu einem Betrieb der Erfassungsvorrichtung (10) nach einem der Ansprüche 1 bis 9.
